(19) Europäisches Patentamt
European Patent Office
Office européen des brevets

(11) Veröffentlichungsnummer: **0 043 535**
**B1**

(12) **EUROPÄISCHE PATENTSCHRIFT**

(45) Veröffentlichungstag der Patentschrift: **31.10.84**

(21) Anmeldenummer: **81105041.8**

(22) Anmeldetag: **30.06.81**

(51) Int. Cl.³: **C 07 D 405/12**, C 07 D 401/12, A 61 K 31/445 // (C07D405/12, 211/58, 311/78), (C07D401/12, 211/58, 221/06)

(54) **Tricyclische Arylether, Verfahren zu ihrer Herstellung sowie diese Verbindungen enthaltende Arzneimittel.**

(30) Priorität: **04.07.80 DE 3025385**

(43) Veröffentlichungstag der Anmeldung: **13.01.82 Patentblatt 82/2**

(45) Bekanntmachung des Hinweises auf die Patenterteilung: **31.10.84 Patentblatt 84/44**

(84) Benannte Vertragsstaaten: **AT BE CH DE FR GB IT LI LU NL SE**

(56) Entgegenhaltungen:
**DE - A - 2 551 235**
**DE - A - 2 735 046**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Winter, Werner, Dr. rer. nat., Mannheimerstrasse 9, D-6148 Heppenheim (DE)**
Erfinder: **Friebe, Walter-Gunar, Dr. rer. nat., Heidelberger Landstrasse 111d, D-6100 Darmstadt (DE)**
Erfinder: **Roesch, Androniki, Dr. med., Werderstrasse 44, D-6800 Mannheim 1 (DE)**
Erfinder: **Wilhelms, Otto-Henning, Dr. rer. nat., Odenwaldstrasse 25/2, D-6941 Weinheim-Rittenweier (DE)**

**EP 0 043 535 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue Arylether-Derivate der allgemeinen Formel I

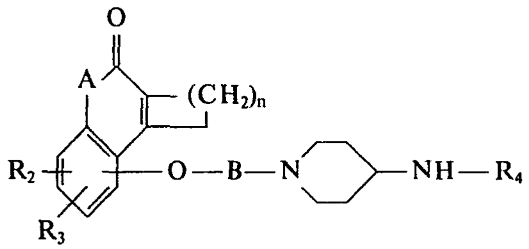

(I)

in der

A ein Sauerstoffatom oder die Gruppe $N-R_1$ darstellt, wobei $R_1$ Wasserstoff oder ein $C_1-C_6$-Alkylrest sein kann,

B einen geradkettigen Alkylenrest mit 2 bis 4 Kohlenstoffatomen,

$R_2$ und $R_3$ die gleich oder verschieden sein können, jeweils Wasserstoff, Halogen, $C_1-C_6$-Alkyl, $C_1-C_4$-Alkoxy, $C_2-C_5$-Alkanoyl oder Hydroxy,

$R_4$ Wasserstoff, einen $C_2-C_7$-Alkanoylrest, der ein- oder mehrfach durch Halogen oder Phenyl substituiert sein kann,
eine gegebenenfalls zweifach durch Methoxy substituierte Cinnamoyl-Gruppe, eine Benzoyl-Gruppe, die ein- oder mehrfach durch Halogen, Hydroxyl, $C_1-C_6$-Alkyl, $C_1-C_6$-Alkoxy, $C_1-C_6$-Alkoxycarbonyl, $C_2-C_7$-Alkanoyloxy, Carboxyl, Nitro, Amino, $C_2-C_7$-Alkanoylamino, Nitril, Trifluormethyl, Carbamoyl, $C_1-C_6$-Alkylthio, $C_1-C_6$-Alkylsulfinyl, $C_1-C_6$-Alkylsulfonyl, $C_2-C_7$-Alkanoyl, Benzoyl, Hydroxy-$C_1-C_6$-alkyl oder $C_1-C_6$-Alkoxy-$C_1-C_6$-alkyl substituiert sein kann,
eine Furancarbonyl-, Thiophencarbonyl- oder Pyridincarbonyl-Gruppe,
eine $C_3-C_7$-Cycloalkyl-carbonyl-Gruppe,
eine Benzolsulfonyl- oder Methansulfonyl-Gruppe oder
ein gegebenenfalls durch $C_1-C_6$-Alkyl oder Phenyl substituierter Carbamoylrest und

n eine ganze Zahl von 1 bis 5

bedeuten, deren pharmakologisch verträgliche Salze.

Sie betrifft ferner Verfahren zu deren Herstellung sowie ihre Verwendung bei der Bekämpfung von allergischen Krankheitsbildern.

Weiterhin betrifft die Erfindung pharmazeutische Präparate mit einem Gehalt an Verbindungen der allgemeinen Formel I sowie die Verwendung von Verbindungen der allgemeinen Formel I zur Herstellung solcher Präparate, wobei die erfindungsgemäßen Substanzen gegebenenfalls in der Form von Salzen nichttoxischer organischer oder anorganischer Säuren oder Basen Anwendung finden.

Verbindungen ähnlicher Konstitution sind in der EP-A-0 013 894 beansprucht. In Fortbildung der dortigen Erfindung wurde nunmehr gefunden, daß die neuen Arylether-Derivate der Formel I, die an Stelle des bicyclischen ein tricyclisches Ringsystem aufweisen, ebenfalls eine ausgeprägte antiallergische Wirkung zeigen, wie sie im pharmakologischen Test der passiven cutanen Anaphylaxie (PCA-Test) in vivo bei Ratten nachgewiesen werden kann. Neben einem starken Antihistamineffekt zeigen sie auch eine antiödematöse und antiphlogistische Wirksamkeit. Die erfindungsgemäßen Verbindungen der allgemeinen Formel I können daher besonders vorteilhaft bei der Bekämpfung von allergischen Krankheiten, beispielsweise von allergischem Asthma, Heuschnupfen und Urticaria verwendet werden.

Die niederen Alkylgruppen der Substituenten $R_1$, $R_2$ und $R_3$ können geradkettig oder verzweigt sein. Als Halogenatome kommen Fluor, Chlor und Brom in Frage.

Der Zahlenwert für n ist vorzugsweise 2, 3 oder 4.

Außer den in den Beispielen genannten Verbindungen sind Gegenstand der vorliegenden Erfindung insbesondere alle Substanzen, die jede mögliche Kombination der in den Beispielen genannten Substituenten aufweisen.

Das erfindungsgemäße Verfahren zur Herstellung der Verbindungen der Formel I ist dadurch gekennzeichnet, daß man in an sich bekannter Weise

eine Verbindung der allgemeinen Formel II

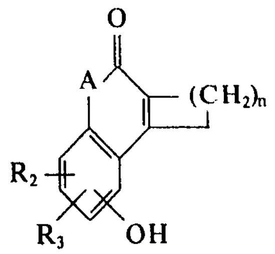

(II)

in der
A, $R_2$, $R_3$ und n die obengenannte Bedeutung haben,
mit einer Verbindung der allgemeinen Formel III

$$X - B - Y \qquad (III)$$

in der
X und Y reaktive Reste bedeuten und B die obengenannte Bedeutung hat,
und einer Verbindung der allgemeinen Formel IV

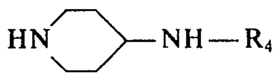

(IV)

in der
$R_4$ die obengenannte Bedeutung hat,
umsetzt,
anschließend gewünschtenfalls die Gruppe $R_4$ durch bekannte Verfahren in eine andere, der obengenannten Bedeutung entsprechende Gruppe $R_4$ umwandelt,
für den Fall, daß $R_1$ Wasserstoff bedeutet, gegebenenfalls nachträglich N-alkyliert
und das erhaltene Reaktionsprodukt gewünschtenfalls in ein pharmakologisch verträgliches Salz überführt.

Als reaktive Reste X und Y der Verbindungen der allgemeinen Formel III kommen Chlor, Brom, Mesyloxy und Tosyloxy in Frage.

Das erfindungsgemäße Verfahren führt man beispielsweise so durch, daß man zunächst Verbindungen der allgemeinen Formel III mit Verbindungen der allgemeinen Formel IV kondensiert und das erhaltene Reaktionsprodukt isoliert. Dieses Zwischenprodukt wird dann mit einer Verbindung der allgemeinen Formel II zur Reaktion gebracht. Die Umsetzung erfolgt zweckmäßig in alkalischem Medium, beispielsweise in einem niederen Alkohol wie z. B. Isopropanol in Anwesenheit von Natriumisopropanolat oder in Dimethylformamid in Anwesenheit von Kaliumcarbonat.

Eine andere Variante besteht darin, zunächst Verbindungen der allgemeinen Formel II mit Verbindungen der allgemeinen Formel III zur Reaktion zu bringen; anschließend wird das erhaltene Reaktionsgemisch mit Verbindungen der allgemeinen Formel IV zum gewünschten Endprodukt der allgemeinen Formel I umgesetzt.

Eine nachträgliche Umwandlung der Gruppe $R_4$ in der allgemeinen Formel I in eine andere Gruppe $R_4$ erfolgt beispielsweise durch Acylierung einer Verbindung der Formel I, in der $R_4$ für Wasserstoff steht, mit einer Verbindung $R_4 - Z$, wobei Z einen reaktiven Rest darstellt. Verbindungen der Formel I mit der Bedeutung $R_4$ = Wasserstoff stellen somit wertvolle Zwischenprodukte zur Herstellung anderer Verbindungen der Formel I dar.

Reaktive Reste Z können alle Reste sein, die in der Peptidchemie zur Aktivierung von Carbonsäuren Verwendung finden, beispielsweise Halogenatome, die Azido-Gruppe, Alkyloxy-, Aryloxy- und Acyloxy-Gruppen.

Die Ausgangsverbindungen der allgemeinen Formeln II, III und IV sind literaturbekannte Substanzen oder können in Analogie zu literaturbekannten Verfahren hergestellt werden.

Die pharmakologisch verträglichen Salze erhält man in üblicher Weise, z. B. durch Neutralisation der Verbindungen der Formel I mit nichttoxischen anorganischen oder organischen Säuren wie z. B. Salzsäure, Schwefelsäure, Phosphorsäure, Bromwasserstoffsäure, Essigsäure, Milchsäure, Zitronensäure, Äpfelsäure, Salicylsäure, Malonsäure, Maleinsäure oder Bernsteinsäure.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z. B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z. B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süßstoffe enthalten. Für die äußerliche Anwendung können die erfindungsgemäßen Substanzen I auch in Form von Pudern und Salben verwendet werden. Sie werden dazu z. B. mit pulverförmigen, physiologisch verträglichen Verdünnungsmitteln bzw. üblichen Salbengrundlagen vermischt.

Die folgenden Beispiele dienen der Erläuterung des Erfindungsgegenstandes.

## Beispiel 1

### 7-[3-(4-Benzamido-piperidino)propoxy]-2,3-dihydro-cyclopenta[c][1]benzopyran-4(1H)on-hydrochlorid

Eine Mischung aus 10,1 g (0,05 mol) 2,3-Dihydro-7-hydroxy-cyclopenta[c][1]benzopyran-4(1H)on, 14,0 g (0,05 mol) 3-(4-Benzamido-piperidino)propylchlorid, 6,9 g (0,05 mol) Kaliumcarbonat und 100 ml Dimethylformamid wird 6 Stunden auf 100°C erhitzt, abgekühlt und filtriert. Nach Waschen mit Wasser wird der Niederschlag (Schmp. 218–220°C) in einer Mischung aus Dichlormethan und Methanol gelöst und durch Zugabe von überschüssiger etherischer Chlorwasserstofflösung in das Hydrochlorid überführt. Man isoliert 14,8 g der Titelverbindung (61% d. Th.) als Hydrochlorid vom Schmp. 274–275°C.

## Beispiele 2 bis 33a

In analoger Weise wie in Beispiel 1 beschrieben erhält man:

| Nr. | Bezeichnung | Ausbeute % | Schmelzpunkt °C |
|---|---|---|---|
| 2 | 2,3-Dihydro-7-{3-[4-(4-fluor-benzamido)piperidino]propoxy}-cyclopenta[c][1]benzopyran-4(1H)-on-hydrochlorid aus 2,3-Dihydro-7-hydroxy-cyclopenta[c][1]benzopyran-4(1H)-on und 3-[4-(4-Fluor-benzamido)piperidino]propylchlorid | 49 | 280–281 |
| 3 | 2,3-Dihydro-7-{3-[4-(4-methoxy-benzamido)piperidino]propoxy}-cyclopenta[c][1]benzopyran-4(1H)-on aus 2,3-Dihydro-7-hydroxy-cyclopenta[c][1]benzopyran-4(1H)-on und 3-[4-(4-Methoxy-benzamido)piperidino]propylchlorid | 80 | 214–215 |
| 4 | 2,3-Dihydro-7-{3-[4-(2-methyl-benzamido)piperidino]propoxy}-cyclopenta[c][1]benzopyran-4(1H)-on aus 2,3-Dihydro-7-hydroxy-cyclopenta[c][1]benzopyran-4(1H)-on und 3-[4-(2-Methyl-benzamido)piperidino]propylchlorid | 46 | 187–188 |
| 5 | 2,3-Dihydro-7-{3-[4-(4-methyl-benzamido)piperidino]propoxy}-cyclopenta[c][1]benzopyran-4(1H)-on-hydrogencarbonat aus 2,3-Dihydro-7-hydroxy-cyclopenta[c][1]benzopyran-4(1H)-on und 3-[4-(4-Methyl-benzamido)piperidino]propylchlorid | 77 | über 280 |
| 6 | 7-{3-[4-(4-t-Butyl-benzamido)piperidino]propoxy}-2,3-dihydro-cyclopenta[c][1]benzopyran-4(1H)-on aus 2,3-Dihydro-7-hydroxy-cyclopenta[c][1]benzopyran-4(1H)-on und 3-[4-(4-t-Butyl-benzamido)piperidino]propylchlorid | 56 | 223–224 |
| 7 | 7-{3-[4-(4-n-Butoxy-benzamido)piperidino]propoxy}-2,3-dihydro-cyclopenta[c][1]benzopyran-4(1H)-on aus 2,3-Dihydro-7-hydroxy-cyclopenta[c][1]benzopyran-4(1H)-on und 3-[4-(4-n-Butoxy-benzamido)piperidino]propylchlorid | 62 | 192–193 |

Fortsetzung

| Nr. | Bezeichnung | Ausbeute % | Schmelzpunkt °C |
|---|---|---|---|
| 8 | 2,3-Dihydro-7-[3-(4-phenylacetamido-piperidino)propoxy]-cyclopenta[c][1]benzopyran-4(1H)-on aus 2,3-Dihydro-7-hydroxy-cyclopenta[c][1]benzopyran-4(1H)-on und 3-(4-Phenylacetamido-piperidino)propylchlorid | 63 | 173–174 |
| 9 | 7-[3-(4-Cyclohexancarboxamido-piperidino)propoxy]-2,3-dihydro-cyclopenta[c][1]benzopyran-4(1H)-on aus 2,3-Dihydro-7-hydroxy-cyclopenta[c][1]benzopyran-4(1H)-on und 3-(4-Cyclohexancarboxamido-piperidino)propylchlorid | 81 | 206–207 |
| 10 | 7-[3-(4-Acetamido-piperidino)propoxy]-2,3-dihydro-cyclopenta[c][1]benzopyran-4(1H)-on aus 2,3-Dihydro-7-hydroxy-cyclopenta[c][1]benzopyran-4(1H)-on und 3-(4-Acetamido-piperidino)propylchlorid | 79 | 182–183 |
| 11 | 7-{3-[4-(4-Acetamido-benzamido)piperidino]propoxy}-2,3-dihydro-cyclopenta[c][1]benzopyran-4(1H)-on aus 2,3-Dihydro-7-hydroxy-cyclopenta[c][1]benzopyran-4(1H)-on und 3-[4-(4-Acetamido-benzamido)piperidino]propylchlorid | 70 | 284–285 |
| 12 | 7-[3-(4-Benzamido-piperidino)propoxy]-2,3-dihydro-6-n-propyl-cyclopenta[c][1]benzopyran-4(1H)-on aus 2,3-Dihydro-7-hydroxy-6-n-propyl-cyclopenta[c][1]benzopyran-4(1H)-on und 3-(4-Benzamido-piperidino)propylchlorid | 61 | 200–201 |
| 13 | 2,3-Dihydro-7-{3-[4-(4-fluor-benzamido)piperidino]propoxy}-6-n-propyl-cyclopenta[c][1]benzopyran-4(1H)-on aus 2,3-Dihydro-7-hydroxy-6-n-propyl-cyclopenta[c][1]benzopyran-4(1H)-on und 3-[4-(4-Fluor-benzamido)piperidino]-propylchlorid | 82 | 210–211 |
| 14 | 6-Acetyl-7-[3-(4-benzamido-piperidino)propoxy]-2,3-dihydro-cyclopenta[c][1]benzopyran-4(1H)-on aus 6-Acetyl-2,3-dihydro-7-hydroxy-cyclopenta[c][1]benzopyran-4(1H)-on und 3-(4-Benzamido-piperidino)propylchlorid | 63 | 218–219 |
| 15 | 7-[3-(4-Benzamido-piperidino)propoxy]-2,3-dihydro-9-hydroxy-cyclopenta[c][1]benzopyran-4(1H)-on aus 2,3-Dihydro-7,9-dihydroxy-cyclopenta[c][1]benzopyran-4(1H)-on und 3-(4-Benzamido-piperidino)propylchlorid | 26 | 235–236 |
| 16 | 3-[3-(4-Benzamido-piperidino)propoxy]-7,8,9,10-tetrahydro-6H-dibenzo[b,d]pyran-6-on-hydrochlorid aus 7,8,9,10-Tetrahydro-3-hydroxy-6H-dibenzo[b,d]-pyran-6-on und 3-(4-Benzamido-piperidino)propylchlorid | 42 | 258–259 |
| 17 | 3-{3-[4-(4-Fluor-benzamido)piperidino]propoxy}-7,8,9,10-tetrahydro-6H-dibenzo[b,d]pyran-6-on-hydrochlorid aus 7,8,9,10-Tetrahydro-3-hydroxy-6H-dibenzo[b,d]pyran-6-on und 3-[4-(4-Fluor-benzamido)piperidino]propylchlorid | 47 | 262–263 |
| 18 | 3-{3-[4-(4-Methoxy-benzamido)piperidino]propoxy}-7,8,9,10-tetrahydro-6H-dibenzo[b,d]pyran-6-on aus 7,8,9,10-Tetrahydro-3-hydroxy-6H-dibenzo[b,d]pyran-6-on und 3-[4-(4-Methoxy-benzamido)piperidino]propylchlorid | 75 | 215–216 |

Fortsetzung

| Nr. | Bezeichnung | Ausbeute % | Schmelzpunkt °C |
|---|---|---|---|
| 19 | 3-{3-[4-(4-Methyl-benzamido)piperidino]propoxy}-7,8,9,10-tetrahydro-6H-dibenzo[b,d]pyran-6-on aus 7,8,9,10-Tetrahydro-3-hydroxy-6H-dibenzo[b,d]pyran-6-on und 3-[4-(4-Methyl-benzamido)piperidino]propylchlorid | 74 | 195–196 |
| 20 | 3-{3-[4-(4-t-Butyl-benzamido)piperidino]propoxy}-7,8,9,10-tetrahydro-6H-dibenzo[b,d]pyran-6-on aus 7,8,9,10-Tetrahydro-3-hydroxy-6H-dibenzo[b,d]pyran-6-on und 3-[4-(4-t-Butyl-benzamido)piperidino]propylchlorid | 60 | 217–218 |
| 21 | 3-{3-[4-(2-Methyl-benzamido)piperidino]propoxy}-7,8,9,10-tetrahydro-6H-dibenzo[b,d]pyran-6-on aus 7,8,9,10-Tetrahydro-3-hydroxy-6H-dibenzo[b,d]pyran-6-on und 3-[4-(2-Methyl-benzamido)piperidino]propylchlorid | 67 | 168–169 |
| 22 | 3-[3-(4-Cyclohexancarboxamido-piperidino)propoxy]-7,8,9,10-tetrahydro-6H-dibenzo[b,d]pyran-6-on aus 7,8,9,10-Tetrahydro-3-hydroxy-6H-dibenzo[b,d]pyran-6-on und 3-(4-Cyclohexancarboxamido-piperidino)propylchlorid | 78 | 207–208 |
| 23 | 3-[3-(4-Acetamido-piperidino)propoxy]-7,8,9,10-tetrahydro-6H-dibenzo[b,d]pyran-6-on aus 7,8,9,10-Tetrahydro-3-hydroxy-6H-dibenzo[b,d]pyran-6-on und 3-(4-Acetamido-piperidino)propylchlorid | 57 | 167–168 |
| 24 | 3-[3-(4-Benzamido-piperidino)propoxy]-4-n-propyl-7,8,9,10-tetrahydro-6H-dibenzo[b,d]pyran-6-on aus 7,8,9,10-Tetrahydro-3-hydroxy-4-n-propyl-6H-dibenzo[b,d]-pyran-6-on und 3-(4-Benzamido-piperidino)propylchlorid | 47 | 191–192 |
| 25 | 3-{3-[4-(4-Fluor-benzamido)piperidino]propoxy}-4-n-propyl-7,8,9,10-tetrahydro-6H-dibenzo[b,d]pyran-6-on aus 7,8,9,10-Tetrahydro-3-hydroxy-4-n-propyl-6H-dibenzo[b,d]-pyran-6-on und 3-[4-(4-Fluor-benzamido)piperidino]propylchlorid | 89 | 207–208 |
| 26 | 3-[3-(4-Benzamido-piperidino)propoxy]-4-methyl-7,8,9,10-tetrahydro-6H-dibenzo[b,d]pyran-6-on aus 7,8,9,10-Tetrahydro-3-hydroxy-4-methyl-6H-dibenzo[b,d]-pyran-6-on und 3-(4-Benzamido-piperidino)propylchlorid | 77 | 224–226 |
| 27 | 4-Acetyl-3-[3-(4-benzamido-piperidino)propoxy]-7,8,9,10-tetrahydro-6H-dibenzo[b,d]pyran-6-on aus 4-Acetyl-7,8,9,10-tetrahydro-3-hydroxy-6H-dibenzo[b,d]-pyran-6-on und 3-(4-Benzamido-piperidino)propylchlorid | 32 | 197–198 |
| 28 | 3-{3-[4-(3-Ethyl-ureido)piperidino]propoxy}-7,8,9,10-tetrahydro-6H-dibenzo[b,d]pyran-6-on aus 7,8,9,10-Tetrahydro-3-hydroxy-6H-dibenzo[b,d]-pyran-6-on und 3-[4-(3-Ethyl-ureido)piperidino]propylchlorid | 72 | 201–202 |
| 29 | 3-[3-(4-Benzamido-piperidino)propoxy]-2-chlor-7,8,9,10-tetrahydro-6H-dibenzo[b,d]pyran-6-on aus 2-Chlor-7,8,9,10-tetrahydro-3-hydroxy-6H-dibenzo[b,d]-pyran-6-on und 3-(4-Benzamido-piperidino)propylchlorid | 83 | 215–216 |
| 30 | 2-[3-(4-Benzamido-piperidino)propoxy]-7,8,9,10-tetrahydro-6H-dibenzo[b,d]pyran-6-on aus 7,8,9,10-Tetrahydro-2-hydroxy-6H-dibenzo[b,d]-pyran-6-on und 3-(4-Benzamido-piperidino)propylchlorid | 69 | 194–195 |

Fortsetzung

| Nr. | Bezeichnung | Ausbeute % | Schmelzpunkt °C |
|---|---|---|---|
| 31 | 6-[3-(4-Benzamido-piperidino)propoxy]-2,3-dihydro-7-methoxy-cyclopenta[c][1]benzopyran-4(1H)-on aus 2,3-Dihydro-6-hydroxy-7-methoxy-cyclopenta[c][1]benzopyran-4(1H)-on und 3-(4-Benzamido-piperidino)propylchlorid | 62 | 156–157 |
| 32 | 3-[3-(4-Benzamido-piperidino)propoxy]-6,7,8,9,10,11-hexahydro-benzo[b]cyclohepta[d]pyran-6-on aus 6,7,8,9,10,11-Hexahydro-3-hydroxy-benzo[b]cyclohepta[d]pyran-6-on und 3-(4-Benzamido-piperidino)propylchlorid | 64 | 185–186 |
| 33 | 7-[3-(4-Benzamido-piperidino)propoxy]-2,3,4,5-tetrahydro-1H-cyclopenta[c]chinolin-4-on aus 2,3,4,5-Tetrahydro-7-hydroxy-1H-cyclopenta[c]-chinolin-4-on und 3-(4-Benzamido-piperidino)propylchlorid | 78 | 273–275 |
| 34 | 3-[3-(4-Phenylacetamido-piperidino)propoxy]-7,8,9,10-tetrahydro-6H-dibenzo[b,d]pyran-6-on aus 7,8,9,10-Tetrahydro-3-hydroxy-6H-dibenzo[b,d]pyran-6-on und 3-(4-Phenylacetamido-piperidino)propylchlorid | 48 | 172–173 |

## Beispiel 35

3-[3-(4-Acetamido-piperidino)propoxy]-7,8,9,10-tetrahydro-6H-dibenzo[b,d]pyran-6-on

Eine Mischung aus 17,3 g (0,051 mol) 3-(3-Brom-propoxy)-7,8,9,10-tetrahydro-6H-dibenzo[b,d]-pyran-6-on, 7,3 g (0,051 mol) 4-Acetamido-piperidin, 5,2 g Triethylamin und 100 ml Tetrahydrofuran wird 14 Stunden zum Rückfluß erwärmt, abgekühlt und filtriert. Man wäscht den Niederschlag mit Tetrahydrofuran und Wasser und trocknet im Vakuum. Es verbleiben 11,3 g der Titelverbindung (55% d. Th.) vom Schmp. 167–168° C, die mit der in Beispiel 23 erhaltenen Substanz identisch ist.

Das als Ausgangsstoff verwendete 3-(3-Brom-propoxy)-7,8,9,10-tetrahydro-6H-dibenzo[b,d]pyran-6-on kann wie folgt erhalten werden:

Zu einer Mischung aus 21,6 g (0,1 mol) 7,8,9,10-Tetrahydro-3-hydroxy-6H-dibenzo[b,d]pyran-6-on, 60,6 g 1,3-Dibrompropan und 150 ml Butanon gibt man bei 75° C innerhalb 3 Stunden 15,2 g (0,11 mol) Kaliumcarbonat, erhitzt 18 Stunden zum Rückfluß, filtriert und engt das Filtrat ein. Man nimmt den Rückstand in Ether auf, wäscht mit verd. Natronlauge, engt die organische Phase ein und verreibt mit Ligroin. Man isoliert 20,5 g der gewünschten Verbindung (61% d. Th.) vom Schmp. 90–92° C.

## Beispiel 36

2,3-Dihydro-7-{3-[4-(4-nitro-benzamido)piperidino]propoxy}-cyclopenta[c][1]benzopyran-4(1H)-on

Eine Mischung aus 4,15 g (0,01 mol) 7-[3-(4-Amino-piperidino)propoxy]-2,3-dihydro-cyclopenta[c][1]benzopyran-4(1H)-on-dihydrochlorid, 2,5 g Natriumhydrogencarbonat und 60 ml Dichlormethan wird bei Eiskühlung mit einer Lösung von 1,86 g (0,01 mol) 4-Nitro-benzoylchlorid in 20 ml Dichlormethan versetzt, 14 Stunden bei Raumtemperatur gerührt, filtriert, der Niederschlag mit Wasser und Dichlormethan gewaschen und durch Chromatographie an Kieselgel (Elutionsmittel Dichlormethan:Methanol 9:1) gereinigt. Man isoliert 2,5 g der Titelverbindung (51% d. Th.) vom Schmp. 212–213° C.

Das als Ausgangsstoff verwendete 7-[3-(4-Amino-piperidino)propoxy]-2,3-dihydro-cyclopenta[c][1]benzopyran-4(1H)-on-dihydrochlorid kann wie folgt erhalten werden:

Eine Mischung aus 76,8 g (0,2 mol) 7-[3-(4-Acetamido-piperidino)propoxy]-2,3-dihydro-cyclopenta[c][1]benzopyran-4(1H)-on (Verbindung aus Beispiel 10), 400 ml Dioxan, 200 ml konz. Salzsäure und 200 ml Wasser wird 8 Stunden zum Rückfluß erhitzt, abgekühlt und filtriert.

Man erhält 49,7 g der gewünschten Verbindung (60% d. Th.) vom Schmp. 225° C.

Beispiele 37 bis 44

In analoger Weise wie in Beispiel 36 beschrieben erhält man:

| Nr. | Bezeichnung | Ausbeute % | Schmelzpunkt °C |
|---|---|---|---|
| 37 | 7-{3-[4-(4-Chlor-benzamido)piperidino]propoxy}-2,3-dihydro-cyclopenta[c][1]benzopyran-4(1H)-on aus 7-[3-(4-Amino-piperidino)propoxy]-2,3-dihydro-cyclopenta-[c][1]benzopyran-4(1H)-on-dihydrochlorid und 4-Chlor-benzoylchlorid | 79 | 213–214 |
| 38 | 2,3-Dihydro-7-{3-[4-(thiophen-2-carboxamido)piperidino]-propoxy}cyclopenta[c][1]benzopyran-4(1H)-on-hydrochlorid aus 7-[3-(4-Amino-piperidino)propoxy]-2,3-dihydro-cyclopenta[c][1]benzopyran-4(1H)-on-dihydrochlorid und Thiophen-2-carbonylchlorid | 64 | 273–274 |
| 39 | 2,3-Dihydro-7-{3-[4-(2-methyl-propionamido)piperidino]-propoxy}cyclopenta[c][1]benzopyran-4(1H)-on aus 7-[3-(4-Amino-piperidino)propoxy]-2,3-dihydro-cyclopenta[c][1]benzopyran-4(1H)-on-dihydrochlorid und 2-Methyl-propionylchlorid | 48 | 186–187 |
| 40 | 3-{3-[4-(4-Chlor-benzamido)piperidino]propoxy}-7,8,9,10-tetrahydro-6H-dibenzo[b,d]pyran-6-on-hydrochlorid aus 3-[3-(4-Amino-piperidino)propoxy]-7,8,9,10-tetrahydro-6H-dibenzo[b,d]pyran-6-on-dihydrochlorid und 4-Chlor-benzoylchlorid | 73 | 268–269 |
| 41 | 3-{3-[4-(5-Chlor-2-methoxy-benzamido)piperidino]propoxy}-7,8,9,10-tetrahydro-6H-dibenzo[b,d]pyran-6-on aus 3-[3-(4-Amino-piperidino)propoxy]-7,8,9,10-tetrahydro-6H-dibenzo[b,d]pyran-6-on-dihydrochlorid und 5-Chlor-2-methoxy-benzoylchlorid | 52 | 170–171 |
| 42 | 3-{3-[4-(Furan-2-carboxamido)piperidino]propoxy}-7,8,9,10-tetrahydro-6H-dibenzo[b,d]pyran-6-on aus 3-[3-(4-Amino-piperidino)propoxy]-7,8,9,10-tetrahydro-6H-dibenzo[b,d]pyran-6-on-dihydrochlorid und Furan-2-carbonylchlorid | 44 | 183–184 |
| 43 | 3-[3-(4-Cyclopropancarboxamido-piperidino)propoxy]-7,8,9,10-tetrahydro-6H-dibenzo[b,d]pyran-6-on aus 3-[3-(4-Amino-piperidino)propoxy]-7,8,9,10-tetrahydro-6H-dibenzo[b,d]pyran-6-on-dihydrochlorid und Cyclopropancarbonylchlorid | 57 | 215–216 |
| 44 | 3-{3-[4-(2-Methyl-propionamido)piperidino]propoxy}-7,8,9,10-tetrahydro-6H-dibenzo[b,d]pyran-6-on aus 3-[3-(4-Amino-piperidino)propoxy]-7,8,9,10-tetrahydro-6H-dibenzo[b,d]pyran-6-on-dihydrochlorid und 2-Methyl-propionylchlorid | 78 | 181–182 |

Beispiel 45

2,3-Dihydro-7-{3-[4-(pyridin-3-carboxamido)piperidino]propoxy}-cyclopenta[c][1]benzopyran-4(1H)-on

Zu einer Mischung aus 1,23 g (0,01 mol) Nicotinsäure, 40 ml Dichlormethan und 1,4 ml Triethylamin tropft man bei −5° C bis −10° C 1,3 ml (0,01 mol) Chlorameisensäureisobutylester, gelöst in 10 ml Dichlormethan. Nach 15 min Rühren bei −10° C fügt man eine Aufschlämmung von 4,15 g (0,01 mol)

7-[3-(4-Amino-piperidino)propoxy]-2,3-dihydro-cyclopenta[c][1]benzopyran-4(1H)-on-dihydrochlorid in 30 ml Dichlormethan und 2,8 g Triethylamin zu, rührt noch 30 min bei −10° C, wäscht mit Wasser, versetzt mit überschüssiger etherischer Chlorwasserstofflösung, filtriert, nimmt den Niederschlag in Wasser auf und stellt mit Natronlauge alkalisch.
Man isoliert 2,8 g der Titelverbindung (62% d. Th.) vom Schmp. 199—200° C.

Beispiele 46 bis 48

In analoger Weise wie in Beispiel 45 beschrieben erhält man:

| Nr. | Bezeichnung | Ausbeute % | Schmelzpunkt °C |
|---|---|---|---|
| 46 | 2,3-Dihydro-7-{3-[4-(3,4-dimethoxy-cinnamoylamido)piperidino]-propoxy}cyclopenta[c][1]benzopyran-4(1H)-on aus 7-[3-(4-Amino-piperidino)propoxy]-2,3-dihydro-cyclopenta[c][1]benzopyran-4(1H)-on-dihydrochlorid und 3,4-Dimethoxy-zimtsäure | 34 | 210—211 |
| 47 | 3-{3-[4-(2-Acetoxy-benzamido)piperidino]propoxy}-7,8,9,10-tetrahydro-6H-dibenzo[b,d]pyran-6-on aus 3-[3-(4-Amino-piperidino)propoxy]-7,8,9,10-tetrahydro-6H-dibenzo[b,d]pyran-6-on-dihydrochlorid und Acetylsalicylsäure | 58 | 176—179 |
| 48 | 2,3-Dihydro-7-{3-[4-(2-nitro-benzamido)piperidino]-propoxy}cyclopenta[c][1]benzopyran-4(1H)-on aus 7-[3-(4-Amino-piperidino)propoxy]-2,3-dihydro-cyclopenta[c][1]benzopyran-4(1H)-on-dihydrochlorid und 2-Nitro-benzoesäure | 45 | 197—198 |

Beispiel 49

2,3-Dihydro-7-{3-[4-(N-phenyl-ureido)piperidino]propoxy}-cyclopenta[c][1]benzopyran-4(1H)-on

Zu der Mischung aus 4,15 g (0,01 mol) 7-[3-(4-Amino-piperidino)propoxy]-2,3-dihydro-cyclopenta[c][1]benzopyran-4(1H)-on-dihydrochlorid, 2,52 g Natriumhydrogencarbonat und 60 ml Dichlormethan tropft man bei 5° C die Lösung von 1,2 g (0,01 mol) N-Phenylisocyanat in 10 ml Dichlormethan, rührt 16 Stunden bei Raumtemperatur, filtriert, wäscht den Niederschlag mit Wasser und Dichlormethan und reinigt durch Chromatographie an Kieselgel (Elutionsmittel Dichlormethan:Methanol 9:1). Man erhält 3,1 g der Titelverbindung (67% d. Th.) vom Schmp. 207—208° C.

Beispiel 50

2,3-Dihydro-7-[3-(4-ureido-piperidino)propoxy]cyclopenta[c][1]benzopyran-4(1H)-on-hydrochlorid

Eine Mischung aus 4,15 g (0,01 mol) 7-[3-(4-Amino-piperidino)propoxy]-2,3-dihydro-cyclopenta[c][1]benzopyran-4(1H)-on-dihydrochlorid, 4,0 g Kaliumcyanat, 50 ml Wasser und 30 ml 2 N Salzsäure wird 3 Tage bei Raumtemperatur gerührt, filtriert und der Niederschlag mit heißem Ethanol extrahiert. Man isoliert 2,4 g der Titelverbindung (57% d. Th.) vom Schmp. 244—245° C.

Beispiel 51

3-{3-[4-(2-Hydroxy-benzamido)piperidino]propoxy}-7,8,9,10-tetrahydro-6H-dibenzo[b,d]pyran-6-on

Eine Mischung aus 5,18 g (0,01 mol) 3-{3-[4-(2-Acetoxy-benzamido)piperidino]propoxy}-7,8,9,10-tetrahydro-6H-dibenzo[b,d]pyran-6-on (Verbindung aus Beispiel 47), 20 ml Dioxan und 20 ml 6 N

Salzsäure wird 10 Stunden zum Rückfluß erhitzt, abgekühlt und filtriert. Man isoliert 3,0 g der Titelverbindung (63% d. Th.) als Hydrochlorid vom Schmp. 232–234° C. Die zugehörige freie Base schmilzt bei 209–211° C.

Beispiel 52

7-{3-[4-(4-Amino-benzamido)piperidino]propoxy}-2,3-dihydro-cyclopenta[c][1]benzopyran-4(1H)-on-dihydrochlorid

In analoger Weise wie in Beispiel 51 beschrieben erhält man durch Verseifung von 7-{3-[4-(4-Acetamido-benzamido)piperidino)propoxy}-2,3-dihydro-cyclopenta[c][1]benzopyran-4(1H)-on (Verbindung aus Beispiel 11) die Titelverbindung nach Chromatographie an Kieselgel (Ausbeute 48% d. Th.) mit dem Schmelzpunkt 267–269° C.

Beispiel 53

7-{3-[4-(2-Amino-benzamido)piperidino]propoxy}-2,3-dihydro-cyclopenta[c][1]benzopyran-4(1H)-on

Eine Lösung von 1,47 g (3 mmol) 2,3-Dihydro-7-{3-[4-(2-nitrobenzamido)piperidino]propoxy}-cyclopenta[c][1]benzopyran-4(1H)-on (Verbindung aus Beispiel 48) in 100 ml Ethanol wird über 1 g Raney-Nickel bei Raumtemperatur und 1 bar Wasserstoffdruck hydriert. Nach Filtration engt man ein und kristallisiert aus Ethanol um. Es verbleiben 0,85 g der Titelverbindung (62% d. Th.) vom Schmp. 269–270° C.

Beispiel 54

Es wurden Tabletten hergestellt: Jede Tablette enthält 10 mg 3-{3-[4-(4-Fluor-benzamido)piperidino]propoxy}-7,8,9,10-tetrahydro-6H-dibenzo[b,d]pyran-6-on-hydrochlorid. Die Tabletten wurden gemäß der folgenden Rezeptur hergestellt:

| | |
|---|---|
| 3-{3-[4-(4-Fluor-benzamido)piperidino]propoxy}-7,8,9,10-tetrahydro-6H-dibenzo[b,d]pyran-6-on-hydrochlorid | 10 g |
| Lactose | 80 g |
| Stärke | 29 g |
| Magnesiumstearat | 1 g |

Vorstehende Verbindung wurde fein pulverisiert und mit Lactose und Stärke vermischt. Das Gemisch wurde in herkömmlicher Weise granuliert. Magnesiumstearat wurde zu dem Granulat gegeben und das Gemisch zu 1000 Tabletten mit einem Einzelgewicht von 0,12 g verpreßt.

**Patentansprüche**

1. Arylether-Derivate der allgemeinen Formel I

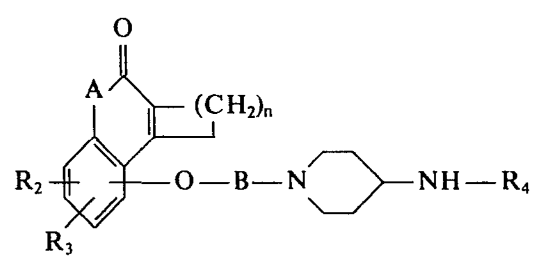

(I)

in der

A ein Sauerstoffatom oder die Gruppe $N-R_1$ darstellt, wobei $R_1$ Wasserstoff oder ein $C_1-C_6$-Alkylrest sein kann,

B einen geradkettigen Alkylenrest mit 2 bis 4 Kohlenstoffatomen,

$R_2$ und $R_3$ die gleich oder verschieden sein können, jeweils Wasserstoff, Halogen, $C_1-C_6$-Alkyl, $C_1-C_4$-Alkoxy, $C_2-C_5$-Alkanoyl oder Hydroxy,

R$_4$ Wasserstoff, einen $C_2-C_7$-Alkanoylrest, der ein- oder mehrfach durch Halogen oder Phenyl substituiert sein kann,
eine gegebenenfalls zweifach durch Methoxy substituierte Cinnamoyl-Gruppe, eine Benzoyl-Gruppe, die ein- oder mehrfach durch Halogen, Hydroxyl, $C_1-C_6$-Alkyl, $C_1-C_6$-Alkoxy, $C_1-C_6$-Alkoxycarbonyl, $C_2-C_7$-Alkanoyloxy, Carboxyl, Nitro, Amino, $C_2-C_7$-Alkanoylamino, Nitril, Trifluormethyl, Carbamoyl, $C_1-C_6$-Alkylthio, $C_1-C_6$-Alkylsulfinyl, $C_1-C_6$-Alkylsulfonyl, $C_2-C_7$-Alkanoyl, Benzoyl, Hydroxy-$C_1-C_6$-alkyl oder $C_1-C_6$-Alkoxy-$C_1-C_6$-alkyl substituiert sein kann,
eine Furancarbonyl-, Thiophencarbonyl- oder Pyridincarbonyl-Gruppe,
eine $C_3-C_7$-Cycloalkyl-carbonyl-Gruppe,
eine Benzolsulfonyl- oder Methansulfonyl-Gruppe oder
ein gegebenenfalls durch $C_1-C_6$-Alkyl oder Phenyl substituierter Carbamoylrest und
n eine ganze Zahl von 1 bis 5

bedeuten, sowie deren pharmakologisch verträgliche Salze.

2. Verbindungen der allgemeinen Formel I gemäß Anspruch 1, in denen B, n, R$_2$, R$_3$ und R$_4$ die angegebenen Bedeutungen haben und A ein Sauerstoffatom bedeutet sowie deren pharmakologisch verträglichen Salze.

3. Verbindungen der allgemeinen Formel I gemäß einem der Ansprüche 1 oder 2, in denen A, B, R$_2$, R$_3$ und R$_4$ die angegebenen Bedeutungen haben und n eine ganze Zahl von 2 bis 4 bedeutet sowie deren pharmakologisch verträglichen Salze.

4. Verbindungen der allgemeinen Formel I gemäß einem der Ansprüche 1, 2 oder 3, in denen A, B, n und R$_4$ die angegebenen Bedeutungen haben und R$_2$ und R$_3$ gleich oder verschieden sein können und Wasserstoff oder einen Acetylrest bedeuten sowie deren pharmakologisch verträglichen Salze.

5. Verbindungen der allgemeinen Formel I gemäß einem der Ansprüche 1, 2, 3 oder 4, in denen A, n, R$_2$, R$_3$ und R$_4$ die angegebenen Bedeutungen haben und B einen n-Propylen-Rest bedeutet sowie deren pharmakologisch verträglichen Salze.

6. Verbindungen der allgemeinen Formel I gemäß einem der Ansprüche 1, 2, 3, 4 oder 5, in denen A, B, n, R$_2$ und R$_3$ die angegebenen Bedeutungen haben und R$_4$ einen gegebenenfalls ein- oder mehrfach durch Halogen, Hydroxyl, $C_1-C_6$-Alkyl, $C_1-C_6$-Alkoxy, $C_1-C_6$-Alkoxycarbonyl, $C_2-C_7$-Alkanoyloxy, Carboxyl, Nitro, Amino, $C_2-C_7$-Alkanoylamino, Nitril, Trifluormethyl, Carbamoyl, $C_1-C_6$-Alkylthio, $C_1-C_6$-Alkylsulfinyl, $C_1-C_6$-Alkylsulfonyl, $C_2-C_7$-Alkanoyl, Benzoyl, Hydroxy-$C_1-C_6$-alkyl oder $C_1-C_6$-Alkoxy-$C_1-C_6$-alkyl substituierten Benzoylrest oder einen Phenylacetylrest bedeutet sowie deren pharmakologisch verträgliche Salze.

7. 6-Acetyl-7-[3-(4-benzamido-piperidino)propoxy]-2,3-dihydro-cyclopenta[c][1]benzopyran-4(1H)-on oder ein pharmakologisch verträgliches Salz.

8. Verfahren zur Herstellung von Arylether-Derivaten der allgemeinen Formel I

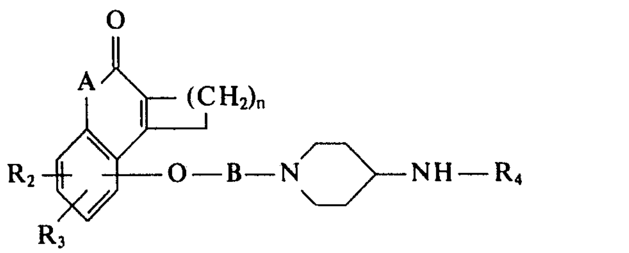

(I)

in der

A ein Sauerstoffatom oder die Gruppe $N-R_1$ darstellt, wobei R$_1$ Wasserstoff oder ein $C_1-C_6$-Alkylrest sein kann,
B einen geradkettigen Alkylenrest mit 2 bis 4 Kohlenstoffatomen,
R$_2$ und R$_3$ die gleich oder verschieden sein können, jeweils Wasserstoff, Halogen, $C_1-C_6$-Alkyl, $C_1-C_4$-Alkoxy, $C_2-C_5$-Alkanoyl oder Hydroxy,
R$_4$ Wasserstoff, einen $C_2-C_7$-Alkanoylrest, der ein- oder mehrfach durch Halogen oder Phenyl substituiert sein kann,
eine gegebenenfalls zweifach durch Methoxy substituierte Cinnamoyl-Gruppe, eine Benzoyl-Gruppe, die ein- oder mehrfach durch Halogen, Hydroxyl, $C_1-C_6$-Alkyl, $C_1-C_6$-Alkoxy, $C_1-C_6$-Alkoxycarbonyl, $C_2-C_7$-Alkanoyloxy, Carboxyl, Nitro, Amino, $C_2-C_7$-Alkanoylamino, Nitril, Trifluormethyl, Carbamoyl, $C_1-C_6$-Alkylthio, $C_1-C_6$-Alkylsulfinyl, $C_1-C_6$-Alkylsulfonyl, $C_2-C_7$-Alkanoyl, Benzoyl, Hydroxy-$C_1-C_6$-alkyl oder $C_1-C_6$-Alkoxy-$C_1-C_6$-alkyl substituiert sein kann,
eine Furancarbonyl-, Thiophencarbonyl- oder Pyridincarbonyl-Gruppe,
eine $C_3-C_7$-Cycloalkyl-carbonyl-Gruppe,

eine Benzolsulfonyl- oder Methansulfonyl-Gruppe oder
ein gegebenenfalls durch $C_1 - C_6$-Alkyl oder Phenyl substituierter Carbamoylrest und

n eine ganze Zahl von 1 bis 5

bedeuten, sowie von deren pharmakologisch verträglichen Salzen, dadurch gekennzeichnet, daß man eine Verbindung der allgemeinen Formel II

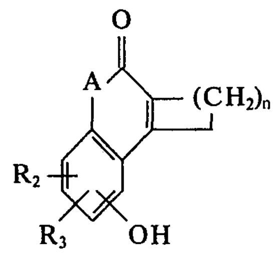

(II)

in der
A, $R_2$, $R_3$ und n die obengenannte Bedeutung haben,
mit einer Verbindung der allgemeinen Formel III

$$X - B - Y \qquad (III)$$

in der
X und Y reaktive Reste bedeuten und B die obengenannte Bedeutung hat,
und einer Verbindung der allgemeinen Formel IV

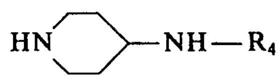

(IV)

in der
$R_4$ die obengenannte Bedeutung hat,
umsetzt,
anschließend gewünschtenfalls die Gruppe $R_4$ durch bekannte Verfahren in eine andere, der obengenannten Bedeutung entsprechende Gruppe $R_4$ umwandelt,
für den Fall, daß $R_1$ Wasserstoff bedeutet, gegebenenfalls nachträglich N-alkyliert
und das erhaltene Reaktionsprodukt gewünschtenfalls in ein pharmakologisch verträgliches Salz überführt.

9. Verbindungen gemäß einem der Ansprüche 1 bis 7 zur Bekämpfung von allergischen Krankheitsbildern.

10. Arzneimittel, gekennzeichnet durch einen Gehalt an Verbindungen der allgemeinen Formel I gemäß einem der Ansprüche 1 bis 7.

## Claims

1. Aryl ether derivatives of the general formula I

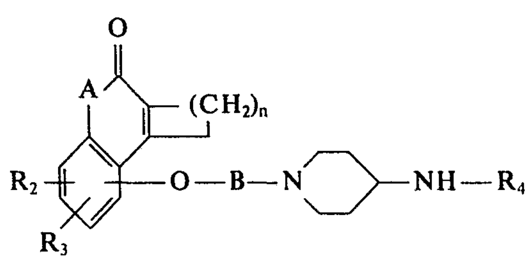

(I)

in which

A represents an oxygen atom or the group $N - R_1$, whereby $R_1$ can be hydrogen or a $C_1 - C_6$-alkyl radical,

B signifies a straight-chained alkylene radical with 2 to 4 carbon atoms,

$R_2$ and $R_3$ which can be the same or different, each signify hydrogen, halogen, $C_1 - C_6$-alkyl, $C_1 - C_4$-alkoxy, $C_2 - C_5$-alkanoyl or hydroxyl,

| | |
|---|---|
| $R_4$ | signifies hydrogen, a $C_2-C_7$-alkanoyl radical, which can be substituted one or more times by halogen or phenyl, a cinnamoyl group optionally substituted twice by methoxy, a benzoyl group, which can optionally be substituted one or more times by halogen, hydroxyl, $C_1-C_6$-alkyl, $C_1-C_6$-alkoxy, $C_1-C_6$-alkoxycarbonyl, $C_2-C_7$-alkanoyloxy, carboxyl, nitro, amino, $C_2-C_7$-alkanoylamino, nitrilo, trifluoromethyl, carbamoyl, $C_1-C_6$-alkylthio, $C_1-C_6$-alkylsulphinyl, $C_1-C_6$-alkylsulphonyl, $C_2-C_7$-alkanoyl, benzoyl, hydroxy-$C_1-C_6$-alkyl or $C_1-C_6$-alkoxy-$C_1-C_6$-alkyl, a furancarbonyl, thiophenecarbonyl or pyridinecarbonyl group, a $C_3-C_7$-cycloalkylcarbonyl group, a benzenesulphonyl or methanesulphonyl group or a carbamoyl radical optionally substituted by $C_1-C_6$-alkyl or phenyl and |
| n | signifies a whole number from 1 to 5, |

as well as their pharmacologically acceptable salts.

2. Compounds of the general formula I according to claim 1, in which B, n, $R_2$, $R_3$ and $R_4$ have the given meanings and A signifies an oxygen atom, as well as their pharmacologically acceptable salts.

3. Compounds of the general formula I according to one of claims 1 or 2, in which A, B, $R_2$, $R_3$ and $R_4$ have the given meanings and n signifies a whole number of 2 to 4, as well as their pharmacologically acceptable salts.

4. Compounds of the general formula I according to one of claims 1, 2 or 3, in which A, B, n and $R_4$ have the given meanings and $R_2$ and $R_3$ can be the same or different and signify hydrogen or an acetyl radical, as well as their pharmacologically acceptable salts.

5. Compounds of the general formula I according to one of claims 1, 2, 3 or 4, in which A, n, $R_2$, $R_3$ and $R_4$ have the given meanings and B signifies an n-propylene radical, as well as their pharmacologically acceptable salts.

6. Compounds of the general formula I according to one of claims 1, 2, 3, 4 or 5, in which A, B, n, $R_2$ and $R_3$ have the given meanings and $R_4$ signifies a benzoyl radical optionally substituted one or more times by halogen, hydroxyl, $C_1-C_6$-alkyl, $C_1-C_6$-alkoxy, $C_1-C_6$-alkoxycarbonyl, $C_2-C_7$-alkanoyloxy, carboxyl, nitro, amino, $C_2-C_7$-alkanoylamino, nitrilo, trifluoromethyl, carbamoyl, $C_1-C_6$-alkylthio, $C_1-C_6$-alkylsulphinyl, $C_1-C_6$-alkylsulphonyl, $C_2-C_7$-alkanoyl, benzoyl, hydroxy-$C_1-C_6$-alkyl or $C_1-C_6$-alkoxy-$C_1-C_6$-alkyl, or a phenylacetyl radical, as well as their pharmacologically acceptable salts.

7. 6-Acetyl-7-[3-(4-benzamidopiperidino)-propoxy]-2,3-dihydrocyclopenta[c][1]benzopyran-4(1H)-one or a pharmacologically acceptable salt thereof.

8. Process for the preparation of aryl ether derivatives of the general formula I

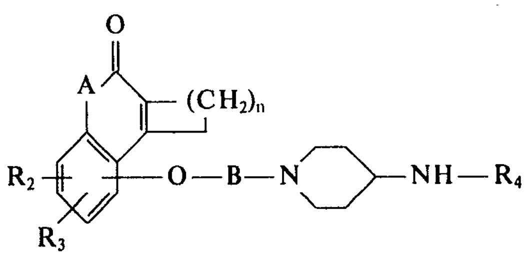

(I)

in which

| | |
|---|---|
| A | represents an oxygen atom or the group $N-R_1$, whereby $R_1$ can be hydrogen or a $C_1-C_6$-alkyl radical, |
| B | signifies a straight-chained alkylene radical with 2 to 4 carbon atoms, |
| $R_2$ and $R_3$ | which can be the same or different, signify hydrogen, halogen, $C_1-C_6$-alkyl, $C_1-C_4$-alkoxy, $C_2-C_5$-alkanoyl or hydroxyl, |
| $R_4$ | signifies hydrogen, a $C_2-C_7$-alkanoyl radical, which can be substituted one or more times by halogen or phenyl, a cinnamoyl group optionally substituted twice by methoxy, a benzoyl group, which can be substituted one or more times by halogen, hydroxyl, $C_1-C_6$-alkyl, $C_1-C_6$-alkoxy, $C_1-C_6$-alkoxycarbonyl, $C_2-C_7$-alkanoyloxy, carboxyl, nitro, amino, $C_2-C_7$-alkanoylamino, nitrilo, trifluoromethyl, carbamoyl, $C_1-C_6$-alkylthio, $C_1-C_6$-alkylsulphinyl, $C_1-C_6$-alkylsulphonyl, $C_2-C_7$-alkanoyl, benzoyl, hydroxy-$C_1-C_6$-alkyl or $C_1-C_6$-alkoxy-$C_1-C_6$-alkyl, a furancarbonyl, thiophenecarbonyl or pyridinecarbonyl group, a $C_3-C_7$-cycloalkylcarbonyl group, |

a benzenesulphonyl or methanesulphonyl group or
a carbamoyl radical optionally substituted by $C_1-C_6$-alkyl or phenyl and

n signifies a whole number from 1 to 5,

as well as of their pharmacologically acceptable salts, characterised in that one reacts a compound of the general formula II

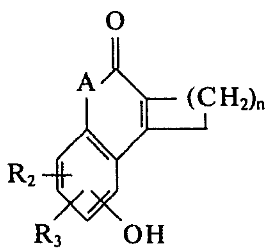

(II)

in which A, $R_2$, $R_3$ and n have the above-mentioned meaning, with a compound of the general formula III

$$X-B-Y \qquad (III)$$

in which X and Y signify reactive residues and B has the above-mentioned meaning, and a compound of the general formula IV

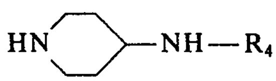

(IV)

in which $R_4$ has the above-mentioned meaning, subsequently possibly converts the group $R_4$ by known processes into another group $R_4$ corresponding to the above-mentioned meaning, for the case in which $R_1$ signifies hydrogen, optionally subsequently N-alkylates and, if desired, converts the reaction product obtained into a pharmacologically acceptable salt.

9. Compounds according to one of claims 1 to 7 for the combating of allergic disease conditions.

10. Medicaments, characterised by a content of compounds of the general formula I according to one of claims 1 to 7.

## Revendications

1. Dérivés d'éthers aryliques de formule générale I:

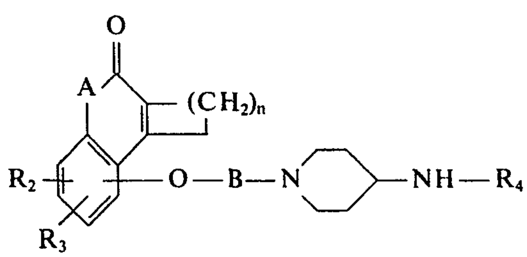

(I)

dans laquelle

A est un atome d'oxygène ou le groupe $N-R_1$ où $R_1$ est de l'hydrogène ou un reste alkyle $C_1-C_6$,

B est un reste alkylène ayant de 2 à 4 atomes de carbone,

$R_2$ et $R_3$ pouvant être identiques ou différents, sont chacun l'hydrogène, un halogène, un alkyle $C_1-C_6$, un alcoxy $C_1-C_4$, un alcanoyle $C_2-C_5$ ou l'hydroxyle,

$R_4$ est l'hydrogène, un reste alcanoyle $C_2-C_7$, pouvant être substitué une ou plusieurs fois par de l'halogène ou du phényle,
un groupe cinnamoyle éventuellement substitué deux fois par méthoxy, un groupe benzoyle pouvant être substitué une ou plusieurs fois par de l'halogène, de l'hydroxyle, de l'alkyle $C_1-C_6$, de l'alcoxy $C_1-C_6$, de l'alcoxycarbonyle $C_1-C_6$, de l'alcanoyloxy $C_2-C_7$, du carboxyle, du nitro, de l'amino, de l'alcanoylamino $C_2-C_7$, du nitrile, du trifluorométhyle, du carbamoyle, de l'alkylthio $C_1-C_6$, de l'alkylsulfinyle $C_1-C_6$, de l'alkylsulfonyle $C_1-C_6$, de l'alcanoyle $C_2-C_7$, du benzoyle, de l'hydroxyalkyle $C_1-C_6$ ou de de l'alcoxy $C_1-C_6$-alkyle $C_1-C_6$, un groupe furannecarbonyle, thiophène carbonyle ou pyridine-carbonyle,

un groupe cyclo-alkyl $C_3 - C_7$-carbonyle
un groupe benzènesulfonyle ou méthane sulfonyle ou
un reste carbamoyle éventuellement substitué par de l'alkyle $C_1 - C_6$ ou du phényle,

n est un nombre entier compris entre 1 et 5,

ainsi que leurs sels pharmacologiquement compatibles.

2. Dérivés de formule générale I selon la revendication 1, dans lesquels B, n, $R_2$, $R_3$ et $R_4$ ont les significations indiquées et A est un atome d'oxygène ainsi que leurs sels pharmacologiquement compatibles.

3. Dérivés de formule générale I selon l'une des revendications 1 ou 2, dans lesquels A, B, $R_2$, $R_3$ et $R_4$ ont les significations indiquées et n est un nombre entier compris entre 2 et 4 ainsi que leurs sels pharmacologiquement compatibles.

4. Dérivés de formule générale I selon l'une des revendications 1, 2 ou 3, dans lesquels A, B, n et $R_4$ ont les significations indiquées et $R_2$ et $R_3$ peuvent être identiques ou différents et sont un hydrogène ou un reste acétyle, ainsi que leurs sels pharmacologiquement compatibles.

5. Dérivés de formule générale I selon l'une des revendications 1, 2, 3 ou 4, dans lesquels A, n, $R_2$, $R_3$ et $R_4$ ont les significations indiquées et B est un reste n-propylène, ainsi que leurs sels pharmacologiquement compatibles.

6. Dérivés de formule générale I selon l'une des revendications 1, 2, 3, 4 ou 5 dans lesquels A, B, n, $R_2$ et $R_3$ ont les significations indiquées et $R_4$ est un reste benzoyle éventuellement substitué une ou plusieurs fois par un halogène, de l'hydroxyle, de l'alkyle $C_1 - C_6$, de l'alcoxy $C_1 - C_6$, de l'alcoxycarbonyle $C_1 - C_6$, de l'alcanoyloxy $C_2 - C_7$, du carboxyle, du nitro, de l'amino, de l'alcanoylamino $C_2 - C_7$, du nitrile, du trifluorométhyle, du carbamoyle, de l'alkylthio $C_1 - C_6$, de l'alkylsulfinyle $C_1 - C_6$, de l'alkylsulfonyle $C_1 - C_6$, de l'alcanoyle $C_2 - C_7$, du benzoyle, de l'hydroxyalkyle $C_1 - C_6$ ou de l'alcoxy $C_1 - C_6$-alkyle $C_1 - C_6$ ou un reste phénylacétyle ainsi que leurs sels pharmacologiquement compatibles.

7. La 6-acétyl-7-[3-(4-benzamido-pipéridino)propoxy]-2,3-dihydro-cyclopenta[c][1]benzopyranne-4(1H)-one ou un sel pharmacologiquement compatible.

8. Procédé pour la préparation de dérivés d'éthers aryliques de formule générale I

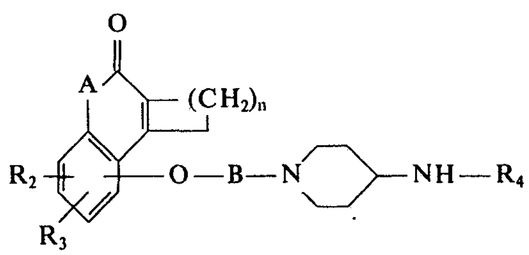

(I)

dans laquelle

A est un atome d'oxygène ou le groupe $N - R_1$ où $R_1$ est de l'hydrogène ou un reste alkyle $C_1 - C_6$,

B est un reste alkylène ayant de 2 à 4 atomes de carbone,

$R_2$ et $R_3$ pouvant être identiques ou différents, sont chacun l'hydrogène, un halogène, un alkyle $C_1 - C_6$, un alcoxy $C_1 - C_4$, un alcanoyle $C_2 - C_5$ ou l'hydroxyle,

$R_4$ est l'hydrogène, un reste alcanoyle $C_2 - C_7$, pouvant être substitué une ou plusieurs fois par de l'halogène ou du phényle,
un groupe cinnamoyle éventuellement substitué deux fois par méthoxy, un groupe benzoyle pouvant être substitué une ou plusieurs fois par de l'halogène, de l'hydroxyle, de l'alkyle $C_1 - C_6$, de l'alcoxy $C_1 - C_6$, de l'alcoxycarbonyle $C_1 - C_6$, de l'alcanoyloxy $C_2 - C_7$, du carboxyle, du nitro, de l'amino, de l'alcanoylamino $C_2 - C_7$, du nitrile, du trifluorométhyle, du carbamoyle, de l'alkylthio $C_1 - C_6$, de l'alkylsulfinyle $C_1 - C_6$, de l'alkylsulfonyle $C_1 - C_6$, de l'alcanoyle $C_2 - C_7$, du benzoyle, de l'hydroxy-alkyle $C_1 - C_6$ ou de l'alcoxy $C_1 - C_6$-alkyle $C_1 - C_6$, un groupe furanne-carbonyle, thiophène-carbonyle ou pyridine-carbonyle,
un groupe cyclo-alkyl $C_3 - C_7$-carbonyle,
un groupe benzènesulfonyle ou méthanesulfonyle ou
un reste carbamoyle éventuellement substitué par de l'alkyle $C_1 - C_6$ ou du phényle,

n est un nombre entier compris entre 1 et 5,

ainsi que leurs sels pharmacologiquement compatibles, caractérisé en ce qu'on fait réagir un composé de formule générale II

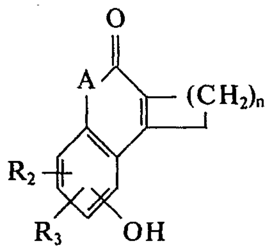

(II)

dans laquelle
A, $R_2$, $R_3$ et n ont la signification ci-dessus indiquée,
avec un composé de formule générale III

$$X - B - Y \qquad (III)$$

dans laquelle
X et Y sont des restes réactifs et B a la signification ci-dessus indiquée,
avec un composé de formule générale IV

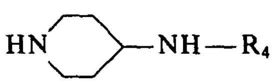

(IV)

dans laquelle $R_4$ a la signification ci-dessus indiquée,
et en ce qu'ensuite on transforme éventuellement le groupe $R_4$ selon un procédé connu en un autre groupe $R_4$ correspondant à la définition ci-dessus indiquée,
on effectue éventuellement ensuite, dans le cas où $R_1$ est de l'hydrogène, une N-alkylation
et on transforme éventuellement le produit de réaction obtenu en un sel pharmacologiquement compatible.

9. Dérivés selon l'une des revendications 1 à 7 pour le traitement de phénomènes pathologiques de type allergique.

10. Médicaments caractérisés en ce qu'ils présentent une teneur en dérivés de formule générale I selon l'une quelconque des revendications 1 à 7.